(19) Europäisches Patentamt
European Patent Office
Office européen des brevets

(11) EP 1 830 898 B1

(12) **FASCICULE DE BREVET EUROPEEN**

(45) Date de publication et mention de la délivrance du brevet:
**05.03.2014 Bulletin 2014/10**

(21) Numéro de dépôt: **05817472.3**

(22) Date de dépôt: **13.12.2005**

(51) Int Cl.:
***A61L 27/16*** *(2006.01)* ***C08F 220/30*** *(2006.01)*

(86) Numéro de dépôt international:
**PCT/EP2005/056732**

(87) Numéro de publication internationale:
**WO 2006/063994 (22.06.2006 Gazette 2006/25)**

(54) **COMPOSITION POLYMERE POUR LENTILLE INTRAOCULAIRE**

POLYMER-ZUSAMMENSETZUNG FÜR EINE INTRAOKULARLINSE

POLYMER COMPOSITION FOR AN INTRAOCULAR LENS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **15.12.2004 BE 200400614**

(43) Date de publication de la demande:
**12.09.2007 Bulletin 2007/37**

(73) Titulaire: **Physiol**
**4031 Angleur (BE)**

(72) Inventeurs:
- **PAGNOULLE, Christophe**
  **B-4800 VERVIERS (BE)**
- **NOLET DE BRAUWERE VAN STEELAND, Marc**
  **B-1640 RHODE-ST-GENESE (BE)**

(74) Mandataire: **Claeys, Pierre et al**
**Gevers**
**Intellectual Property House**
**Holidaystraat 5**
**1831 Diegem (BE)**

(56) Documents cités:
**EP-A- 0 898 972**
**US-A1- 2002 007 032**
**US-A1- 2002 049 290**
**US-B1- 6 245 106**
**US-B1- 6 353 069**

- **PATENT ABSTRACTS OF JAPAN vol. 011, no. 086 (M-572), 17 mars 1987 (1987-03-17) & JP 61 238614 A (KIKUSUI KAGAKU KOGYO KK), 23 octobre 1986 (1986-10-23)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention est relative à une composition polymère pour lentille intraoculaire, comprenant :

- au moins un premier monomère (méth)acrylique hydrophile,
- au moins un second monomère hydrophobe à indice de réfraction élevé, copolymérisable avec ledit au moins un premier monomère,
- un agent de réticulation desdits premier et second monomères, et
- un amorceur de copolymérisation,

dans laquelle lesdits premier et second monomères représentent ensemble au moins 90 % en poids de la composition globale.

On connaît depuis un certain temps des matières pliables appropriées pour être utilisées dans des lentilles intraoculaires artificielles.

On connaît une composition polymère de ce genre (voir par exemple US-2002/0049290). Cette demande de brevet concerne des hydrogels à indice de réfraction élevé, d'environ 1,45 ou plus, qui sont formés d'un polymère réticulé préparé à partir d'un monomère hydrophile et d'un monomère à indice de réfraction élevé, hydrophobe, le monomère hydrophile étant présent en une quantité supérieure à celle du monomère hydrophobe en vue d'augmenter au maximum sa teneur en eau après hydratation. Ces types d'hydrogels absorbent une grande quantité d'eau, par exemple jusqu'à 20 à 70 % en poids. Ils sont rigides lorsque la composition est anhydre et ils deviennent souples et pliables à l'état hydraté, ce qui en fait des produits très favorables par rapport aux autres matériels du point de vue de l'usinage à la température ambiante, d'une part, et de la facilité d'implantation dans l'oeil, d'autre part. Cependant étant donné que l'indice de réfraction de l'eau est bas, c'est-à-dire de 1,33, une addition d'eau à la composition polymère diminue l'indice de réfraction de la composition à l'état hydraté par rapport à celui de son état anhydre. D'autre part les hydrogels à haute teneur en eau semblent accélérer la migration des cellules épithéliales sur la surface des lentilles et donc produire une opacification capsulaire ultérieure.

On connaît aussi des compositions acryliques dites à faible température de transition vitreuse (Tg) qui présentent typiquement un indice de réfraction élevé (> 1,50). La composition acrylique est choisie de façon à présenter, à l'état anhydre, une température de transition vitreuse quelque peu inférieure à la température de l'oeil (environ 34°C) de façon que la lentille montre une rigidité (module de Young) à la température du corps qui reflète les caractéristiques d'un cuir. Ces compositions comprennent d'une manière générale une copolymérisation de deux monomères hydrophobes réticulés (voir US-5.290.892). On connaît aussi des compositions à base de monomères hydrophobes de ce genre et d'une petite quantité d'un troisième monomère de caractère hydrophile reconnu en plus de l'agent de réticulation classique et de l'amorceur de polymérisation (voir EP-0898972 et US-6.353.069). Ces dernières compositions réduisent le risque de réflexions lumineuses ou scintillements (glistening), dus à la formation de micro-vacuoles d'eau au sein du matériau, qui apparaissent généralement dans les compositions acryliques à faible température de transition vitreuse précédemment citées.

Ces compositions acryliques présentent à l'état anhydre une température de transition vitreuse relativement basse, généralement quelque peu en dessous de la température ambiante. La fabrication des lentilles qui s'effectue sur les compositions à l'état sec et peuvent demander un fraisage classique et des techniques d'usinage au tour doivent être effectuées à une température bien en dessous de la température ambiante, ce qui complique ces opérations et les rend relativement coûteuses.

Par monomères hydrophobes, il faut entendre suivant l'invention les monomères donnant lieu à des homopolymères insolubles dans l'eau. De même, un monomère est dit, suivant l'invention, hydrophile dans la mesure où les homopolymères correspondants sont au moins partiellement solubles dans l'eau.

La présente invention a pour but de mettre au point une composition polymère pour lentille intraoculaire qui présente un indice de réfraction relativement élevé, de bonnes propriétés d'implantation et d'usinage et donc un équilibre approprié entre les propriétés antagonistes de flexibilité mécanique et de facilité à la fabrication à l'aide d'un tour.

On a résolu ces problèmes par une composition polymère telle qu'indiquée au début, qui contient 29 à 39% en poids dudit au moins un premier monomère hydrophibe et 53 à 63 % en poids dudit au moins un second monomère hydrophobe, et dans laquelle l'agent de réticulation est un polymère hydrophobe qui présente plusieurs groupes éthyléniquement insaturés polymérisables et qui comprend un nombre d'unités de base (degré de polymérisation) égal ou supérieur à 5. Par polymères, il faut entendre suivant l'invention des substances constituées de grandes molécules formées par la répétition d'un même motif composé d'une ou de plusieurs unités de base (monomère).

Comme premier monomère on peut envisager tout monomère acrylique ou méthacrylique hydrophile approprié, comme par exemple ceux indiqués dans la demande de brevet précitée US 2002/0049290. De préférence on fait usage d'un méthacrylate, en particulier, mais non de manière limitative, du méthacrylate de 2-hydroxyéthyle (HEMA).

Comme second monomère à indice de réfraction élevé, on envisage en particulier des monomères présentant

un indice de réfraction supérieur à 1,5. Comme monomères de ce genre, on utilise des composés hydrophobes, de préférence aromatiques. On envisage notamment des monomères choisis parmi le groupe constitué des acrylates de phénoxyéthyle, des acrylates de phénoxypolyéthylène glycol, des acrylates de phényle et des acrylates d'alkyle. De préférence on fait usage d'acrylate de 2-phénoxyéthyle (EGPEA) ou d'acrylate de phénoxypolyéthylène glycol (PEGPEA).

L'agent de réticulation est un polymère hydrophobe qui présente plusieurs groupes éthyléniquement insaturés polymérisables et qui comprend un nombre d'unités de base égal ou supérieur à 5, de préférence compris entre 5 et 10, très avantageusement entre 6 et 8. Cet agent de réticulation présente de ce fait un poids moléculaire relativement élevé et il est utilisé à une concentration d'au moins 5 % en poids par rapport à la composition globale. Les agents de réticulation suivant l'invention peuvent par exemple consister en polypropylène glycol portant deux insaturations polymérisables. L'agent de réticulation peut répondre à la formule I

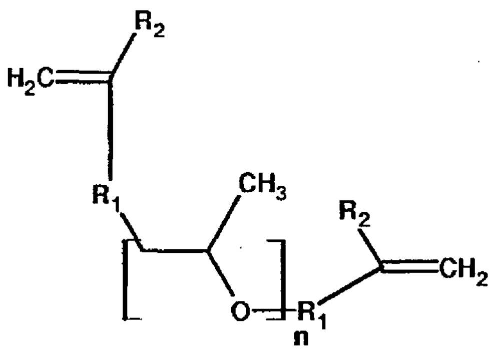

dans laquelle

$R_1$ représente une simple liaison ou un groupe -O- ou -$X_1$-CO-$X_2$-où $X_1$ et $X_2$, représentent, indépendamment l'un de l'autre, une simple liaison ou un groupe -O- ou -NH-,
$R_2$ représente H ou -$CH_3$, et
n est un nombre tel que le poids moléculaire moyen pondéral de l'agent de réticulation soit de 400 à 850.

Les agents de réticulation correspondant à la formule I sont commercialement disponibles ou ils peuvent être synthétisés selon des procédés bien connus de l'homme de métier. Les monomères représentatifs de la formule I présentent avantageusement un poids moléculaire moyen pondéral de 400 à 700, de préférence de 500 à 600. On peut utiliser d'autres agents de réticulation hydrophobes en variante comme ceux présentant un squelette de polytétraméthylène glycol. L'utilisation d'un agent de réticulation de poids moléculaire relativement élevé permet d'obtenir un copolymère ayant un pourcentage en poids relativement élevé d'agent de réticulation, tout en maintenant une faible densité de réticulation. Il en résulte que les copolymères suivant l'invention évitent l'introduction d'une densité excessive de réticulation qui rend les matières inflexibles et fragiles. Par exemple, la concentration d'agent de réticulation suivant l'invention préférée, supérieure à 5 % en poids, par exemple de 8 % en poids, donne lieu à une composition présentant une flexibilité et une élasticité améliorées, sans représenter un inconvénient pour l'usinabilité par les techniques conventionnelles au tour. Au contraire, lorsque des agents de réticulation classiques de faible poids moléculaire sont introduits à la même concentration en poids à l'intérieur d'une formulation de monomères, on obtient une composition qui devient rigide, fragile et impliable. L'agent de réticulation de haut poids moléculaire suivant la présente invention peut donc remplacer à la fois les agents de réticulation conventionnels et un troisième monomère, qui est parfois utilisé pour ajuster les propriétés finales de flexibilité, d'absorption d'eau, de Tg (voir par exemple EP-0898972 et US-6.353.069). De plus, l'incorporation d'un pourcentage élevé d'un agent de réticulation à poids moléculaire élevé dans la formulation de monomères augmente sa viscosité. Une solution analogue à du sirop est obtenue, ce qui permet un meilleur contrôle des tubes (carottes) ou disques polymérisés.

Une copolymérisation desdits premier et second monomères suivant l'invention par l'agent de réticulation tel que prévu ci-dessus permet d'obtenir une composition qui présente une teneur en eau à saturation faible, c'est-à-dire inférieure à 10% en poids de la composition globale, une température de transition vitreuse inférieure à la température de l'oeil, en particulier de l'ordre de 15 à 30°C, de préférence de 20-25°C, à un état anhydre et inférieure ou égale à 15°C après hydratation à saturation, ainsi qu'un indice de réfraction au moins égal à 1,5.

Il était inattendu que des matières acryliques à faible température de transition vitreuse puissent être polymérisées en présentant simultanément une teneur en eau modérée après hydratation, une flexibilité maximale à l'état hydraté, et une usinabilité améliorée en partant uniquement de deux monomères principaux. Habituellement on considérait que la flexibilité d'une composition de lentille intraoculaire était fonction de la teneur en eau de celle-ci et que l'indice de réfraction était inversement proportionnel à la teneur en eau. D'une manière inattendue on a trouvé que, dans la composition suivant l'invention, la flexibilité du copolymère est à présent contrôlée de manière basique par la température de transition vitreuse de la matière, indépendamment de la teneur en eau. Lorsque la teneur en second monomère augmente, la température de transition vitreuse à l'état hydraté diminue et on peut ainsi l'ajuster en dessous de la température ambiante. D'autre part, la flexibilité du polymère est augmentée, en dépit de la teneur en eau faible à saturation. D'une manière inattendue la flexibilité du polymère est inversement proportionnelle à la teneur en eau, l'indice de réfraction restant inversement proportionnel à celle-ci également.

Comme amorceur de copolymérisation, on peut utiliser n'importe quel amorceur approprié courant, connu de l'homme de métier.

La présente invention concerne également l'utilisation d'une composition suivant l'invention pour la fabrication d'une lentille intraoculaire ainsi qu'une lentille intraoculaire obtenue à l'aide de la composition suivant l'invention.

Les lentilles produites à l'aide d'une composition suivant l'invention présentent donc une teneur en eau modérée, un indice de réfraction relativement élevé et un équilibre approprié inattendu entre les propriétés antagonistes de flexibilité mécanique et de facilité de fabrication par des techniques de tournage et de fraisage. Grâce aux températures de transition vitreuse que la composition suivant l'invention présente à l'état anhydre, les lentilles possèdent des caractéristiques de dureté suffisantes à la température ambiante pour pouvoir subir des processus de découpe au tour conventionnels dans des conditions de refroidissement modérées. Par conditions de refroidissement modérées il faut entendre que la pièce n'est pas exposée, comme c'est le cas dans l'état de la technique, à un jet d'azote liquide ou d'air refroidi pendant le découpage et qu'elle ne nécessite pas d'être auparavant congelée dans un dispositif de refroidissement. D'autre part, après hydratation de la lentille qui, à l'état anhydre, ne présente pas les caractéristiques de flexibilité appropriées pour être pliée, la température de transition vitreuse chute brusquement d'au moins 10°C par rapport à la composition anhydre. Il en résulte une flexibilité maximale à la température ambiante et en particulier à la température du corps lorsque les lentilles à l'état hydraté doivent être implantées dans l'oeil.

L'invention va à présent être expliquée de manière plus détaillée à l'aide d'exemples non limitatifs.

**Exemple 1**

On combine du méthacrylate de 2-hydroxyéthyle (HEMA) et de l'acrylate de 2-phénoxyéthyle (EGPEA) en différents rapports pondéraux en présence de 0,2 % en poids d'amorceur (azobis(isobutyronitrile) (AIBN)) et de 2,9 % en poids d'un agent de réticulation conventionnel à base de diméthacrylate d'éthylène glycol (EGDMA). Le volume total de chaque composition est d'environ 14 ml. Après homogénéisation dans un bain ultrasonique pendant 10 minutes à la température ambiante, chaque solution est filtrée et transférée dans un flacon pourvu d'un agitateur magnétique. Ensuite, sous agitation, on fait barboter dans chaque solution du gaz argon ultrapur pendant 2 minutes et on les soumet ensuite à un vide dynamique pendant 2 minutes. Le cycle barbotage à l'argon/vide est répété au moins 3 fois. 2 ml de la solution sont ensuite transférés dans des moules précédemment saturés d'argon par l'utilisation d'une seringue. Le moule rempli est alors placé dans un bain d'eau à température régulée et il est chauffé à 58°C pendant au moins 72 heures. Après cette étape de polymérisation initiale, le moule est lentement refroidi à 50°C et maintenu à cette température pendant au moins 20 minutes. Le moule est ouvert et les pièces moulées sont cuites dans un four sous vide à successivement 65°C pendant 2 heures, 75°C pendant 2 heures, 90°C pendant 12 heures et 120°C pendant 12 heures. On procède ensuite à un lent refroidissement à la température ambiante tout en maintenant le vide statique. Les parties centrales des pièces moulées sont ensuite découpées en disques d'une épaisseur de 3,0 mm par une technique conventionnelle de coupe au tour avec la condition que, pour les compositions à faibles températures de transition vitreuse, cette partie soit maintenue à une température nettement en dessous de la température ambiante en l'exposant à un jet d'air refroidi. Les composés potentiellement toxiques, dont les monomères résiduels, sont extraits pendant 48 h avec de l'isopropanol à reflux dans un Soxhlet. Les disques sont ensuite transférés dans un four pendant 48 heures à la température ambiante. Les disques acryliques partiellement séchés sont placés dans un four sous vide et chauffés à successivement 45°C pendant 24 heures et 75°C pendant 48 heures pour compléter le processus de séchage. Les disques séchés sont ensuite entreposés sous atmosphère réduite avec un agent dessiccant. Les disques sont alors placés dans des flacons individuels en verre remplis de 20 ml d'eau désionisée et ils sont hydratés à la température ambiante pendant quelques jours jusqu'à ce qu'ils atteignent une masse constante. L'indice de réfraction de chaque échantillon hydraté est déterminé sur un réfractomètre. Pour déterminer la teneur en eau à l'équilibre, les disques hydratés sont séchés dans une étuve à 75°C, sous une atmosphère réduite, jusqu'à obtention d'une masse constante. La teneur en eau à l'équilibre pour chaque disque est déterminée par gravimétrie en utilisant l'équation suivante :

$$teneur\ en\ eau\ à\ l'équilibre = \left( \frac{(masse\ (hydratée) - masse\ (déshydratée))}{masse\ (déshydratée)} \right) \times 100$$

La température de transition vitreuse de chaque disque est mesurée par des calorimétries à balayage différentiel (DSC) avec une rampe de 10°C par minute et elle est déterminée au point central de la transition de la courbe du flux thermique.

La dureté Shore A des disques hydratés et non hydratés a été déterminée à la température ambiante avec un duromètre, à 15 secondes si cela n'est pas spécifié.

La figure 1 représente un graphique dans lequel la teneur en eau à l'équilibre (% en poids), l'indice de réfraction à l'état hydraté, la température de transition vitreuse à l'état sec et la dureté Shore A à la fois à l'état hydraté et à l'état déshydraté sont indiqués en fonction de la concentration en % en poids du monomère hydrophile HEMA.

Comme attendu, les compositions ayant une teneur élevée en HEMA présentent une teneur en eau à l'équilibre élevée. Par conséquent celle-ci est proportionnelle au pourcentage en poids du monomère hydrophile, tandis que l'indice de réfraction apparaît inversement proportionnel à la concentration en monomère hydrophile et donc à la teneur en eau à l'équilibre. D'une manière attendue, la température de transition vitreuse à l'état anhydre croît lorsque la concentration en monomère à indice de réfraction élevé, c'est-à-dire EGPEA, diminue par rapport à celle du monomère hydrophile.

Pour les compositions riches en HEMA, la température de transition vitreuse (Tg) à l'état anhydre est toujours supérieure à la température ambiante. D'une manière générale, la flexibilité des spécimens dans cette gamme de compositions riches en HEMA n'est pas affectée par le rapport HEMA/EGPEA et le disque reste rigide et non pliable avec des valeurs de dureté Shore A supérieures à 90. Après hydratation, la flexibilité des spécimens est remarquablement améliorée en étant principalement contrôlée par les teneurs en eau. Par conséquent la dureté Shore A après hydratation devient inversement proportionnelle à la teneur en HEMA ou à la teneur en eau à l'équilibre. Cependant, pour que la flexibilité soit suffisante pour l'application visée, c'est-à-dire une dureté Shore A inférieure à 60, la composition doit contenir au minimum une teneur critique en HEMA, c'est-à-dire 75 % en poids, et donc présenter une teneur en eau à saturation supérieure à 15 % en poids. Ces compositions tombent dans la catégorie des hydrogels et elles présentent l'inconvénient d'indices de réfraction limités, c'est-à-dire inférieurs à 1,5.

Pour les compositions riches en EGPEA, on a découvert d'une manière inattendue que la flexibilité des spécimens, que ce soit avant ou après l'hydratation, augmente au fur et à mesure qu'augmente la teneur en EGPEA. Ici au contraire de ce qui se passait pour les compositions riches en HEMA, la dureté Shore A n'est pas proportionnelle à la teneur en HEMA et elle est légèrement diminuée par absorption d'eau. Pour cette gamme de compositions, la flexibilité est principalement contrôlée par la température de transition vitreuse (Tg) des spécimens plutôt que par la teneur en eau. Donc, au-delà d'une quantité critique d'EGPEA, c'est-à-dire 50 % en poids, la flexibilité des spécimens est remarquablement augmentée au fur et à mesure de l'augmentation de la concentration en EGPEA. Il y a une gamme de compositions EGPEA/HEMA où l'effet plastifiant des molécules d'eau d'hydratation sur la flexibilité peut être rendue maximum tout en maintenant les indices de réfraction au-dessus de 1,5 et en conservant une teneur en eau à l'équilibre à une valeur modérée. La présence d'eau dans les matières est hautement souhaitable pour inhiber la formation de scintillements (glistenings) dus à la formation de micro-vacuoles d'eau au sein du matériau. En effet, pour des teneurs en HEMA supérieures à 10 % en poids et inférieures à 30 % en poids, la dureté Shore A après hydratation est inférieure à sa valeur critique pour la capacité de pliage requise, c'est-à-dire 60, l'indice de réfraction est supérieur à 1,5 et la teneur en eau est supérieure à 5 % en poids. Cependant, dans cette gamme de compositions, les températures de transition vitreuse à l'état anhydre sont inférieures à la température ambiante, c'est-à-dire que tous ces spécimens sont difficiles à usiner au tour. De plus, les disques souffrent d'un manque d'élasticité en présentant une déformation plastique, ce qui rend ce type de matière acrylique à faible température de transition vitreuse peu souhaitable.

## Exemple 2

Quelques mélanges de monomères sont polymérisés de la même manière que décrit dans l'exemple 1, à l'exception du fait que certaines compositions comprennent comme agent de réticulation, au lieu du EGDMA conventionnel, un agent de réticulation polymère hydrophobe à poids moléculaire relativement élevé, c'est-à-dire dans cet exemple du diméthacrylate de polypropylène glycol (PPGDMA, poids moléculaire d'environ 560).

Le tableau 1 ci-dessous montre les quantités des monomères polymérisables et de l'agent de réticulation. Pour maintenir la densité plus ou moins constante de la réticulation, la teneur en poids de PPGDMA a été ajustée à 8 % en poids (par rapport à la composition globale) contre 2,9 % en poids de EGDMA de poids moléculaire limité. Les copolymères synthétisés ont été découpés en disques de 3 mm d'épaisseur comme décrit dans l'exemple 1. Ainsi qu'il ressort du tableau 2 ci-dessous, l'utilisation de l'agent de réticulation suivant l'invention plutôt que du monomère conventionnel (voir exemple 2.1) augmente d'une manière remarquable la flexibilité de la composition, comme indiqué par les valeurs

de dureté Shore A. Cette situation est valable quelle que soit la base de comparaison après hydratation, l'élasticité à température ambiante, c'est-à-dire à 22°C, est remarquablement améliorée et la dureté Shore A (mesurée à 15 secondes) décroît de façon significative avec l'absorption d'eau. Cette amélioration de l'élasticité est mise en lumière par le fait que la matière hydratée de la présente invention atteint plus rapidement la valeur Shore A d'équilibre que ne le fait la forme non hydratée.

Au contraire des autres compositions de comparaison présentant une flexibilité élevée (voir exemples de comparaison 2.5 et 2.6) et une faible teneur en eau, la température de transition vitreuse à l'état anhydre de la composition suivant l'invention est maintenue à une valeur élevée inattendue, proche de la température ambiante, ce qui rend cette composition suffisamment dure pour être aisément usinée par des techniques de tournage conventionnelles. Par conséquent la composition suivant la présente invention montre un équilibre optimal entre les propriétés antagonistes de flexibilité et d'usinabilité. Après hydratation, la température de transition vitreuse tombe à 9°C, ce qui rend cette composition hautement pliable à la température ambiante et même à une température plus basse. Les disques polymérisés sont homogènes et exempts d'un quelconque défaut que ce soit des fissures ou des bulles.

**Tableau 1**

| Exemples (*) | EGPEA (% poids) | HEMA (% poids) | EGPEA/HEMA (poids/ poids) | PPGDMA (560 daltons) (% poids) | EGDMA (% poids) |
|---|---|---|---|---|---|
| 2.1 Présente invention | 57,9 | 34 | 1,7 | 8 | - |
| 2.2 Ex. Comp. | 61,1 | 36 | 1,7 | - | 2,9 |
| 2.3 Ex. Comp. | 57,9 | 39,2 | 1,5 | - | 2,9 |
| 2.4 Ex. Comp. | 63,1 | 34 | 1,85 | - | 2,9 |
| 2.5 Ex. Comp. | 69 | 28,1 | 2,45 | - | 2,9 |
| 2.6 Ex. Comp. | 78,6 | 18,5 | 4,25 | - | 2,9 |

(*) Tous les exemples ci-dessus contiennent en outre 0,2 % en poids d'AIBN et 0,5 % en poids de BHPEA.
EGPEA = acrylate de 2-phénoxyéthyle
HEMA = méthacrylate de 2-hydroxyéthyle
PPGDMA = diméthacrylate de polypropylèneglycol
EGDMA = diméthacrylate d'éthylèneglycol
BHPEA = acrylate de 2-(4-benzoyl-3-hydroxyphénoxy)éthyle
AIBN = 2,2'-azobis(isobutyronitrile)

**Tableau 2**

| Exemples | Tg (°C) anhydre | Shore A (hydraté) | Teneur en eau à l'équilibre (% poids) | Indice de réfraction |
|---|---|---|---|---|
| 2.1 Présente invention | (*) 23 ± 1 | 49 ± 2 | 6,5 ± 0,25 | ~1,525 |
| 2.2 Ex. Comp. | 21 ± 1 | 66 ± 4 | 7,5 ± 0,3 | ~1,525 |
| 2.3 Ex. Comp. | 23 ± 1 | 67 ± 3 | 8 ± 0,3 | ~1,522 |
| 2.4 Ex. Comp. | 20 ± 1 | 65 ± 3 | 7 ± 0,3 | ~1,53 |
| 2.5 Ex. Comp. | 17 ± 1 | 58 ± 3 | 5,5 ± 0,3 | ~1,54 |
| 2.6 Ex. Comp. | 13 ± 1 | 46 ± 2 | 4 ± 0,1 | ~1,55 |

(*) Tg (hydraté) = 9 ± 1 °C.

## Exemple 3

On réalise diverses compositions à base de deux monomères et d'un agent de réticulation de la même manière que décrit dans l'exemple 1. Les compositions polymérisées sont indiquées dans le tableau 3 ci-dessous où les valeurs sont indiquées en % en poids de la quantité totale de la composition. Les copolymères synthétisés ont été découpés

en disques d'une épaisseur de 3 mm comme dans l'exemple 1. Ces disques sont ensuite usinés en lentilles biconvexes (25,0 D). Une technique de découpe au tour est utilisée, mais le disque est fixé sur une plaque de fixation rotative (broche) par un très mince film d'eau congelée. La plaque de fixation a été préalablement pulvérisée par de l'eau qui a été congelée par un fluide refroidi par un tube vortex utilisant de l'air comprimé. En utilisant cette technique, la cire, qui est habituellement utilisée pour fixer le disque sur la broche, n'est pas nécessaire et la plaque de fixation est maintenue à une gamme de température basse pendant tout le processus de découpe. Le spécimen est donc maintenu à une température suffisamment basse pour être utilisé sans être exposé à de l'azote liquide ou un jet d'air refroidi pendant la découpe. Les lentilles sont alors placées dans des flacons individuels remplis de 20 ml d'eau désionisée et elles sont hydratées à la température ambiante pendant quelques jours jusqu'à ce que l'on atteigne une masse constante.

La transparence (évaluation des "glistenings") est mesurée conformément au test suivant. Le flacon contenant la lentille usinée dans l'eau désionisée est placé dans un four à circulation d'air préchauffé à 45°C. Les échantillons sont maintenus dans le four pendant 48 heures avant d'être refroidis à 6°C pendant 24 heures. On les laisse atteindre l'équilibre de la température ambiante pendant 2 heures avant analyse. La lentille est ensuite retirée du flacon et elle est placée sur une lame microscopique. L'observation des "glistenings" est faite par un microscope à un grossissement de 50-150 x. Un échantillon est qualifié exempt de scintillement, lorsqu'aucune micro-vacuole d'eau n'est détectée dans la lentille jusqu'à un grossissement de 200 x. Ainsi qu'il apparaît clairement sur le tableau 4 ci-dessous, seule la composition suivant l'invention (exemple 3.1) montre une combinaison idéale d'absence de scintillement, de compatibilité à la stérilisation à la vapeur et de capacité d'usinage, en plus des propriétés de flexibilité maximum rapportées dans l'exemple précédent.

**Tableau 3**

| Exemples | Monomère à IR élevé | % poids | Monomère | % poids | Agent de réticulation | % poids | | |
|---|---|---|---|---|---|---|---|---|
| 3.1 Présente invention | EGPEA | (57,9) | HEMA | (34) | PPGDMA | (8) | | |
| 3.2 Ex. Comp. | EGPEA | (100) | | | | | | |
| 3.3 Ex. Camp. | PEA | (65) | PEMA | (32) | BDDA | (3) | | |
| 3.4 Ex. Comp. | EGPEA | (93) | BPDMA | (7) | | | | |
| 3.5 Ex. Comp. | EMA | (35) | TFEMA | (10) | BA (ou EA) | (53) | EGDMA | (2,5) |
| 3.6 Ex. Comp. | EGPEA | (57,9) | HEMA | (34) | PEGDMA | (8) | | |

(*) Tous les exemples ci-dessus contiennent en outre 0,2 % en poids d'AIBN et 0,5 % en poids de BHPEA.
PEA = acrylate de phényléthyle
EMA = méthacrylate d'éthyle
PEMA = méthacrylate de phényléthyle
BPDMA = diméthacrylate de bisphénol A éthoxylé
TFEMA = méthacrylate de 2,2,2-trifluoroéthyle
BDDA = diméthacrylate de butanediol
BA = acrylate de butyle
EA = acrylate d'éthyle
PEGDMA = diméthacrylate de polyélhylène glycol.

**Tableau 4**

| Exemples | Tg (°C) anhydre | Teneur en eau à l'équilibre (% poids) | "Glistenings" | Stérilisation à la vapeur (1) | Usinable (2) |
|---|---|---|---|---|---|
| 3.1 Présente invention | 23 | 6,5 | non | OK | Oui |
| 3.2 Ex. Comp. | 5 | <2 | Intenses | Opaque | Non |
| 3.3 Ex. Comp. | 19 | <2 | Intenses | Opaque | Oui |
| 3.4 Ex. Comp. | 8 | <2 | Très intenses (opaque) | Opaque | Non |
| 3.5 Ex. Comp. | 12 | <2 | Intenses | Opaque | Difficile |
| 3.6 Ex. Comp. | Ce matériau est opaque après hydratation à température ambiante et impossible à tourner ou fraiser | | | | |
| (1) A 121 °C pendant 30 minutes.<br>(2) Standards d'évaluation :<br>Oui : sans exposer la pièce à un jet d'azote liquide ou d'air refroidi<br>Difficile : sans cette exposition, à moins de refroidir la pièce à -21°C pendant au moins 5 heures.<br>Non : impossible selon la procédure de l'exemple 3. | | | | | |

Il doit être entendu que la présente invention n'est en aucune façon limitée aux formes de réalisation décrites ci-dessus et que bien des modifications peuvent y être apportées sans sortir du cadre des revendications annexées.

## Revendications

1. Composition polymère pour lentille intraoculaire, comprenant :

   - au moins un premier monomère (méth)acrylique hydrophile,
   - au moins un second monomère hydrophobe à indice de réfraction élevé, copolymérisable avec ledit au moins un premier monomère,
   - un agent de réticulation desdits premier et second monomères, et
   - un amorceur de copolymérisation, dans laquelle lesdits premier et second monomères représentent ensemble au moins 90 % en poids de la composition globale,

   **caractérisée en ce qu'**elle contient 29 à 39% en poids dudit au moins un premier monomère hydrophile et 53 à 63% en poids dudit au moins un second monomère hydrophobe, et
   **en ce que** l'agent de réticulation est un polymère hydrophobe qui présente plusieurs groupes éthyléniquement insaturés polymérisables et qui comprend un nombre d'unités de base (degré de polymérisation) égal ou supérieur à 5.

2. Composition suivant la revendication 1, **caractérisée en ce que** qu'elle comprend au moins 5 % en poids d'agent de réticulation par rapport à la composition globale.

3. Composition suivant l'une ou l'autre des revendications 1 et 2, **caractérisée en ce que** l'agent de réticulation répond à la formule I

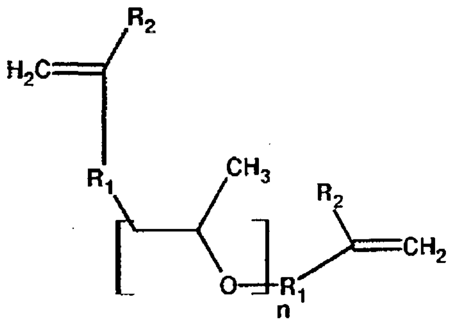

dans laquelle

$R_1$ représente une simple liaison ou un groupe -O- ou $-X_1-CO-X_2-$, où $X_1$ et $X_2$ représentent, indépendamment l'un de l'autre, une simple liaison ou un groupe -O- ou -NH-,
$R_2$ représente H ou $-CH_3$, et
n est un nombre tel que le poids moléculaire moyen pondéral de l'agent de réticulation soit de 400 à 850.

**4.** Composition suivant l'une quelconque des revendications 1 à 3, **caractérisée en ce que** ledit au moins un premier monomère hydrophile est un méthacrylate.

**5.** Composition suivant l'une quelconque des revendications 1 à 4, **caractérisée en ce que** ledit au moins un second monomère présente un indice de réfraction supérieur à 1,5.

**6.** Composition suivant la revendication 5, **caractérisé en ce que** ledit au moins un second monomère est un composé aromatique.

**7.** Composition suivant l'une ou l'autre des revendications 5 et 6, **caractérisée en ce que** ledit au moins un second monomère est choisi parmi le groupe constitué des acrylates de phénoxyéthyle, des acrylates de phénoxypolyéthylèneglycol, des acrylates de phényle et des acrylates d'alkyle.

**8.** Composition suivant l'une quelconque des revendications 1 à 7, **caractérisée en ce qu'**elle présente, après hydratation, une teneur en eau à saturation inférieure à 10 % en poids de la composition globale.

**9.** Composition suivant l'une quelconque des revendications 1 à 8, **caractérisée en ce qu'**elle présente, à un état anhydre, une température de transition vitreuse inférieure à la température d'un oeil.

**10.** Composition suivant la revendication 9, **caractérisée en ce qu'**elle présente à cet état anhydre une température de transition vitreuse de l'ordre de 15 à 30°C, de préférence de 20 à 25°C.

**11.** Composition suivant l'une quelconque des revendications 1 à 10, **caractérisée en ce qu'**elle présente, après hydratation, une température de transition vitreuse inférieure ou égale à 15°C à saturation.

**12.** Composition suivant l'une quelconque des revendications 1 à 11, **caractérisée en ce qu'**elle présente un indice de réfraction au moins égal à 1,5.

**13.** Utilisation d'une composition suivant l'une quelconque des revendications 1 à 12, pour la fabrication d'une lentille intraoculaire.

**14.** Lentille intraoculaire à base d'une composition suivant l'une quelconque des revendications 1 à 12.

**Patentansprüche**

**1.** Polymer-Zusammensetzung für eine Intraokularlinse, umfassend:

- mindestens ein erstes hydrophiles (Meth)acrylmonomer,

- mindestens ein zweites hydrophobes Monomer mit hohem Brechungsindex, das mit dem mindestens einen ersten Monomer copolymerisierbar ist,
- ein Mittel zur Vernetzung des ersten und des zweiten Monomers, und
- einen Copolymerisationsinitiator,

wobei das erste und das zweite Monomer zusammen mindestens 90 Gew.-% der Gesamtzusammensetzung bilden, **dadurch gekennzeichnet, dass** sie 29 bis 39 Gew.-% des mindestens einen ersten hydrophilen Monomers und 53 bis 63 Gew.-% des mindestens einen zweiten hydrophoben Monomers enthält, und dadurch, dass das Mittel zur Vernetzung ein hydrophobes Polymer ist, das mehrere polymerisierbare, ethylenisch ungesättigte Gruppen aufweist und das eine Anzahl von Basiseinheiten (Polymerisationsgrad) von gleich oder größer 5 umfasst.

2. Zusammensetzung nach Anspruch 1, **dadurch gekennzeichnet, dass** sie mindestens 5 Gew.-% des Vernetzungsmittels, bezogen auf die Gesamtzusammensetzung, umfasst.

3. Zusammensetzung nach irgendeinem der Ansprüche 1 und 2, **dadurch gekennzeichnet, dass** das Vernetzungsmittel der folgenden Formel I entspricht:

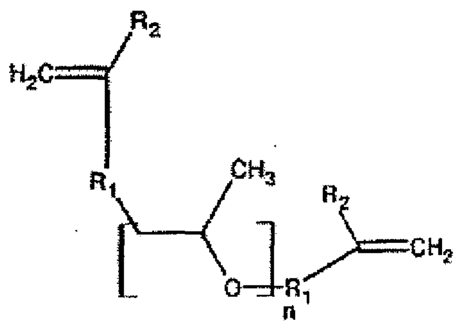

wobei

$R_1$ für eine Einfachbindung oder eine Gruppe -O- oder -$X_1$CO-$X_2$-steht, worin $X_1$ und $X_2$ unabhängig voneinander für eine Einfachbindung oder eine Gruppe -O- oder -NH- stehen,
$R_2$ für H oder-$CH_3$ steht, und
n eine solche Zahl ist, dass das gewichtsgemittelte Molekulargewicht des Vernetzungsmittels 400 bis 850 beträgt.

4. Zusammensetzung nach einem der Ansprüche 1 bis 3, **dadurch gekennzeichnet, dass** das mindestens eine erste hydrophile Monomer ein Methacrylat ist.

5. Zusammensetzung nach einem der Ansprüche 1 bis 4, **dadurch gekennzeichnet, dass** das mindestens eine zweite Monomer einen Brechungsindex von größer als 1,5 aufweist.

6. Zusammensetzung nach Anspruch 5, **dadurch gekennzeichnet, dass** das mindestens eine zweite Monomer eine aromatische Verbindung ist.

7. Zusammensetzung nach irgendeinem der Ansprüche 5 und 6, **dadurch gekennzeichnet, dass** das mindestens eine zweite Monomer ausgewählt ist aus der Gruppe bestehend aus Phenoxyethylacrylaten, Phenoxypolyethylenglycolacrylaten, Phenylacrylaten und Alkylacrylaten.

8. Zusammensetzung nach einem der Ansprüche 1 bis 7, **dadurch gekennzeichnet, dass** sie nach der Hydratation einen Sättigungswassergehalt von weniger als 10 Gew.-% der Gesamtzusammensetzung aufweist.

9. Zusammensetzung nach einem der Ansprüche 1 bis 8, **dadurch gekennzeichnet, dass** sie in wasserfreiem Zustand eine Glasübergangstemperatur aufweist, die unterhalb der Temperatur eines Auges liegt.

10. Zusammensetzung nach Anspruch 9, **dadurch gekennzeichnet, dass** sie in diesem wasserfreien Zustand eine

Glasübergangstemperatur in der Größenordnung von 15 bis 30 °C, bevorzugt von 20 bis 25 °C aufweist.

**11.** Zusammensetzung nach einem der Ansprüche 1 bis 10, **dadurch gekennzeichnet, dass** sie nach der Hydratation bei Sättigung eine Glasübergangstemperatur von kleiner oder gleich 15 °C aufweist.

**12.** Zusammensetzung nach einem der Ansprüche 1 bis 11, **dadurch gekennzeichnet, dass** sie einen Brechungsindex von mindestens gleich 1,5 aufweist.

**13.** Verwendung einer Zusammensetzung nach einem der Ansprüche 1 bis 12 zur Herstellung einer Intraokularlinse.

**14.** Intraokularlinse auf Basis einer Zusammensetzung nach einem der Ansprüche 1 bis 12.

## Claims

**1.** A polymer composition for intraocular lens, comprising:

- at least one first hydrophilic (meth)acrylic monomer;
- at least one second hydrophobic monomer with high refractive index, copolymerisable with said at least one first monomer;
- a cross-linking agent of said first and second monomers; and
- a copolymerisation initiator,

wherein the said first and second monomers together represent at least 90 weight % of the global composition, **characterized in that** it contains 29 to 39 weight % of said at least one first hydrophilic monomer and 53 to 63 weight % of said at least one second hydrophobic monomer, and **in that** the cross-linking agent is a hydrophobic polymer having several polymerisable, ethylenically unsaturated groups and comprising a number of base units (degree of polymerisation) of 5 or more.

**2.** The composition according to claim 1, **characterized in that** it comprises at least 5 weight % of cross-linking agent relative to the global composition.

**3.** The composition according to either of claims 1 and 2, **characterized in that** the cross-linking agent meets formula 1:

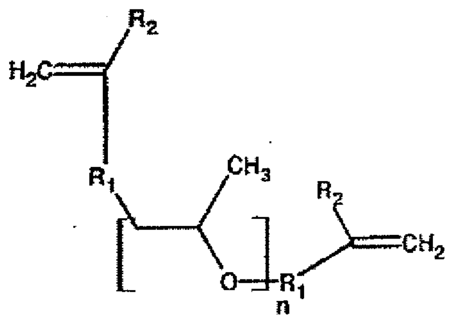

where:

$R_1$ is a single bond or -O- or $-X_1-CO-X_2-$ group in which $X_1$ and $X_2$ independently of each other represent a single bond or -0- or -NH- group;
$R_2$ represents H or $-CH_3$; and
n is a number such that the weight average molecular weight of the cross-linking agent is 400 to 850.

**4.** The composition according to any of claims 1 to 3, **characterized in that** the said at least one first hydrophilic monomer is a methacrylate

**5.** The composition according to any of claims 1 to 4, **characterized in that** the said at least one second monomer has a refractive index higher than 1.5.

**6.** The composition according to claim 5, **characterized in that** the said at least one second monomer is an aromatic compound.

**7.** The composition according to either of claims 5 and 6, **characterized in that** the said at least one second monomer is chosen from the group formed by phenoxyethyl acrylates, phenoxypolyethylene glycol acrylates, phenyl acrylates and alkyl acrylates.

**8.** The composition according to any of claims 1 to 7 **characterized in that**, after hydration, it has a saturated water content lower than 10 weight % of the global composition.

**9.** The composition according to any of claims 1 to 8, **characterized in that** in the anhydrous state it has a glass transition temperature lower than the temperature of an eye.

**10.** The composition according to claim 9, **characterized in that** in this anhydrous state it has a glass transition temperature of the order of 15 to 30°C, preferably 20 to 25°C.

**11.** The composition according to any of claims 1 to 10 **characterized in that**, after hydration, it has a glass transition temperature equal to or lower than 15°C at saturation.

**12.** The composition according to any of claims 1 to 11, **characterized in that** it has a refractive index of at least 1.5.

**13.** The use of a composition according to any of claims 1 to 12 to produce an intraocular lens.

**14.** An intraocular lens containing a composition according to any of claims 1 to 12.

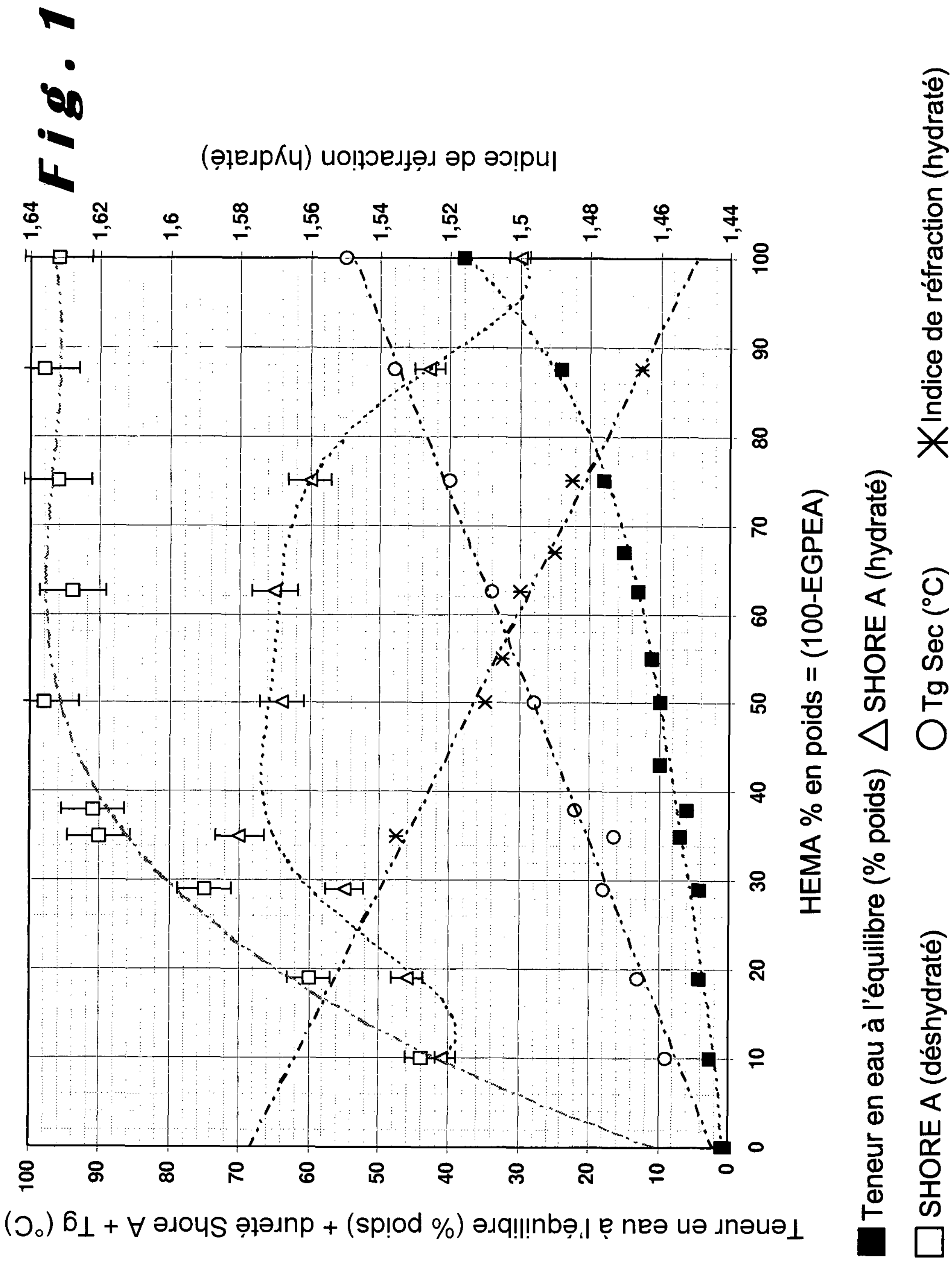
Indice de réfraction (hydraté)
1,64
1,62
1,6
1,58
1,56
1,54
1,52
1,5
1,48
1,46
1,44
Teneur en eau à l'équilibre (% poids) + dureté Shore A + Tg (°C)
100
90
80
70
60
50
40
30
20
10
0
HEMA % en poids = (100-EGPEA)
Teneur en eau à l'équilibre (% poids)
SHORE A (hydraté)
SHORE A (déshydraté)
Tg Sec (°C)
Indice de réfraction (hydraté)

Fig. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20020049290 A
- US 5290892 A
- EP 0898972 A
- US 6353069 B